(19) Europäisches Patentamt
European Patent Office
Office européen des brevets

(11) Veröffentlichungsnummer: **0 214 566 B1**

# (12) EUROPÄISCHE PATENTSCHRIFT

(45) Veröffentlichungstag der Patentschrift: **13.03.91**

(51) Int. Cl.$^{5}$: **C07D 213/50**, C07D 213/30, A01N 43/40

(21) Anmeldenummer: **86111948.5**

(22) Anmeldetag: **29.08.86**

## (54) 3-Propenylpyridine und ihre Verwendung als Fungizide.

(30) Priorität: **05.09.85 DE 3531659**

(43) Veröffentlichungstag der Anmeldung:
**18.03.87 Patentblatt 87/12**

(45) Bekanntmachung des Hinweises auf die Patenterteilung:
**13.03.91 Patentblatt 91/11**

(84) Benannte Vertragsstaaten:
**AT BE CH DE FR GB IT LI NL SE**

(56) Entgegenhaltungen:
**EP-A- 0 074 018**
**FR-A- 2 047 883**

**CHEMICAL ABSTRACTS, vol. 60, no. 4, 17th February 1964, Spalte 4046, Columbus, Ohio, US; A.C. ANNIGERI et al.: "2'-, 3'-, and 4'-Azachalcones and their bacteriostatic activity"**

(73) Patentinhaber: **BASF Aktiengesellschaft**
**Carl-Bosch-Strasse 38**
**W-6700 Ludwigshafen(DE)**

(72) Erfinder: **Buschmann, Ernst, Dr.**
**Georg-Ludwig-Krebs-Strasse 10**
**W-6700 Ludwigshafen(DE)**
Erfinder: **Goetz, Norbert, Dr.**
**Schoefferstrasse 25**
**W-6520 Worms 1(DE)**
Erfinder: **Sproesser, Linhard, Dr.**
**Goethestrasse 4**
**W-6702 Bad Duerkheim(DE)**
Erfinder: **Ammermann, Eberhard, Dr.**
**Sachsenstrasse 3**
**W-6700 Ludwigshafen(DE)**
Erfinder: **Pommer, Ernst-Heinrich, Dr.**
**Berliner Platz 7**
**W-6703 Limburgerhof(DE)**

**EP 0 214 566 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft neue Pyridinderivate und deren Salze sowie fungizide Mittel, die diese Verbindungen enthalten und Verfahren zu ihrer Herstellung.

Fungizide Pyridinderivate, beispielsweise 3-(4-Chlorphenyl)-1-(3--pyridinyl)-2-propen-1-on werden beschrieben in Bull. Soc. Chim. Fr., 7 - 8, Pt. 2, 1427 - 32 (1974). Die fungizide Wirkung dieser Pyridine ist unbefriedigend.

Es wurde nun gefunden, daß Pyridine der Formel I

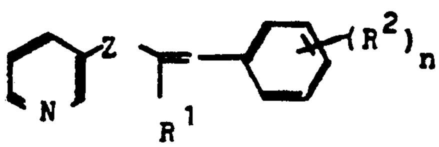

I,

in der

$R^1$ für Alkyl, Cycloalkyl, gegebenenfalls substituiertes Phenyl

$R^2$ für Wasserstoff, Halogen, Alkoxy, Alkyl, Phenyl,

n für die Zahlen 1, 2, 3,

Z für C = 0 oder CHOH stehen

und die pflanzenverträglichen Salze dieser Verbindungen eine gute fungizide Wirkung haben, die der fungiziden Aktivität der bekannten Pyridinderivate überlegen ist.

$R^1$ bedeutet Alkyl mit 1 bis 8 C-Atomen z.B. Methyl, Ethyl, n-Propyl, Isopropyl, n-Butyl, Isobutyl, tert.-Butyl, n-Pentyl, Isopentyl, n-Hexyl, n-Heptyl, n-Octyl; Cycloalkyl mit 3 bis 7 C-Atomen z.B. Cyclopropyl, Cyclopentyl, Cyclohexyl, Cycloheptyl, Cyclooctyl; gegebenenfalls substituiertes Phenyl z.B. Phenyl, Halogenphenyl, 4-Cl-Phenyl, 2,4-$Cl_2$-Phenyl, 4-F-Phenyl, $C_1$-$C_4$-Alkyl-phenyl, 4-$CH_3$-Phenyl, Diphenyl, 4-$C_6H_5$-Phenyl, Nitrophenyl, 4-$NO_2$-Phenyl, $C_1$-$C_4$-Alkoxyphenyl, 4-$OCH_3$-Phenyl.

$R^2$ bedeutet Wasserstoff, Fluor, Chlor, Brom, Jod, Alkoxy mit 1 - 5 C-Atomen z.B. Methoxy, Ethoxy, n-Propoxy, iso-Propoxy, n-Butoxy, tert.-Butoxy; Alkyl mit 1 - 5 C-Atomen z.B. Methyl, Ethyl, n-Propyl, iso-Propyl, n-Butyl, tert.-Butyl, iso-Pentyl; Phenyl.

Pflanzenphysiologisch verträgliche Salze der Verbindungen sind z.B. Salze mit Halogenwasserstoffsäuren, Hydrobromide, Hydrochloride; Sulfate, Phosphate; Salze mit Alkylcarbonsäuren, Oxalate, Acetate, Formiate oder Additionsverbindungen mit Säuren von Tensiden (z.B. Dodecylbenzolsulfonsäure). Die Verbindungen können hinsichtlich der C = C-Doppelbindung als Z oder E-Isomere oder Isomerengemische vorliegen. Sowohl die einzelnen Isomere als auch ihre Mischungen werden von der Erfindung umfaßt.

Das Verfahren zur Herstellung der neuen Verbindungen ist dadurch gekennzeichnet, daß man ein Pyridylketon der Formel II

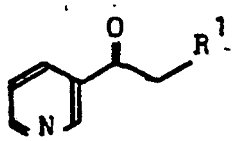

II,

in der $R^1$ die in Anspruch 1 genannten Bedeutungen hat, in Gegenwart eines inerten Lösungsmittels wie Methanol, Ethanol, Essigester, Dimethoxyethan, Toluol oder auch ohne Lösungsmittel in Gegenwart einer Base (NaOH, NaOMe, KOH, Piperidin) oder in Gegenwart einer Lewis-Säure z.B. ZnO oder $B_2O_3$ umsetzt mit einem Benzaldehyd der Formel III

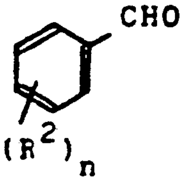

III,

in der $R^2$ und n die in Anspruch 1 genannten Bedeutungen haben. Die Reaktionstemperatur liegt dabei zwischen 5O und 2OO° C. Je nach Lösungsmittel wird die Reaktion unter Normaldruck oder unter erhöhtem

Druck durchgeführt. Dabei werden die Pyridylketone der Formel IV

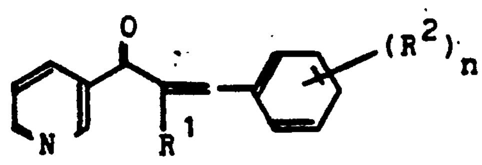

IV

erhalten, in der $R^1$, $R^2$ und n die in Anspruch 1 genannten Bedeutungen haben.

Die Ketone IV können gegebenenfalls zu den Carbinolen der Formel V, in der $R^1$, $R^2$ und n die in Anspruch 1 genannten Bedeutungen haben, reduziert werden. Geeignete Reduktionsmittel hierfür sind z.B. $NaBH_4$, $LiAlH_4$ oder $H_2$ + Katalysator.

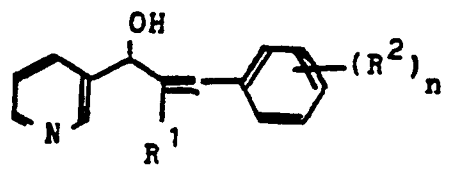

V

Die folgenden Beispiele erläutern die Herstellung der neuen fungiziden Pyridine:

Beispiel 1

2-n-Butyl-3-(2,4-dichlorbenzyl)-1-pyridin-3-yl-2-propen-1-on

In einem 3 l-Autoklaven wird eine Mischung aus 1,5 kg Pyridin-3-yl-n-pentylketon, 3OO g 2,4-Dichlorbenzaldehyd und 59 g Bortrioxid zunächst 5 h lang auf 15O° C bei 7O bar Druck und anschließend noch 5 h auf 18O° C bei 75 bar Druck erhitzt. Nach Abtrennung des Bortrioxids erfolgt die destillative Reinigung des Rohrproduktes. Ausbeute 316 g der Verbindung Nr. 1, kp 19O° C (O,1 mbar).

Beispiel 2

2-n-Butyl-3-(2,4-dichlorbenzyl)-1-pyridin-3-yl-2-propen-1-ol

Zu 32 g der Verbindung Nr. 1, gelöst in 5OO ml $CH_3OH$, werden portionsweise 7,3 g $NaBH_4$ zugegeben. Man rührt 1 h unter Rückfluß, gibt 1 l $H_2O$ zu und extrahiert mit $CH_2Cl_2$. Nach Trocknen über $Na_2SO_4$ und Einengen ergibt die Destillation 2O g der Verbindung Nr. 2, Sdp 2O9° C O,2 mbar.

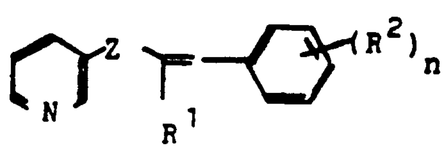

| Verb. Nr. | $R^1$ | $(R^2)_n$ | Z | Sdp °C/mbar |
|---|---|---|---|---|
| 1 | n-Butyl | 2,4-$Cl_2$ | C=O | 190/0.1 |
| 2 | n-Butyl | 2,4-$Cl_2$ | CHOH | 209/0.2 |
| 3 | n-Propyl | 2,4-$Cl_2$ | C=O | |
| 4 | n-Propyl | 2,4-$Cl_2$ | CHOH | |
| 5 | n-Pentyl | 2,4-$Cl_2$ | C=O | |
| 6 | n-Pentyl | 2,4-$Cl_2$ | CHOH | |
| 7 | neo-Pentyl | 2,4-$Cl_2$ | C=O | |
| 8 | neo-Pentyl | 2,4-$Cl_2$ | CHOH | |
| 9 | $C_6H_5$ | 2,4-$Cl_2$ | C=O | |
| 10 | $C_6H_5$ | 2,4-$Cl_2$ | CHOH | |
| 11 | Benzyl | 2,4-$Cl_2$ | C=O | |
| 12 | Benzyl | 2,4-$Cl_2$ | CHOH | |
| 13 | n-Butyl | H | C=O | |
| 14 | n-Butyl | H | CHOH | |
| 15 | n-Butyl | 4-Cl | C=O | 188/0.2 |

| Verb. Nr. | $R^1$ | $(R^2)_n$ | Z | Sdp °C/mbar |
|---|---|---|---|---|
| 16 | n-Butyl | 4-Cl | CHOH | 206-210/0.2 |
| 17 | n-Butyl | 4-F | C=O | 170-180/0.05 |
| 18 | n-Butyl | 4-F | CHOH | 170-180/0.3 |
| 19 | n-Butyl | 4-$CH_3$ | C=O | 170-184/0.05 |
| 20 | n-Butyl | 4-$CH_3$ | CHOH | 176-180/0.2 |
| 21 | n-Butyl | 4-$OCH_3$ | C=O | 170/0.05 |
| 22 | n-Butyl | 4-$OCH_3$ | CHOH | 216/0.8 |
| 23 | n-Butyl | 4-tert.-Butyl | C=O | 160/0.01 |
| 24 | n-Butyl | 4-tert.-Butyl | CHOH | 190-2/0.2 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum an Kürbisgewächsen,
Podosphaera leucotricha an Äpfeln,
Uncinula necator an Reben,
Puccinia-Arten an Getreide,

Rhizoctonia solani an Baumwolle,
Ustilago-Arten an Getreide und Zuckerrohr,
Venturia inaequalis (Schorf) an Äpfeln,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Hemileia vastatrix an Kaffee,
Alternaria solani an Kartoffeln, Tomaten
Plasmopara viticola an Reben sowie Fusarium- und Verticillium-Arten an verschiedenen Pflanzen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle, (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz u.a. zur Bekämpfung holzzerstörender Pilze wie Coniophora puteanea und Polystictus versicolor eingesetzt werden. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweise durch Versprühen, Vernebeln, Verstäuben, Verstreuen, Beizen oder Gießen.

Beispiele für solche Zubereitungen sind:

I. Man vermischt 90 Gewichtsteile der Verbindung Nr. 15 mit 10 Gewichtsteilen N-Methyl-alpha-pyrrolidon und erhält eine Lösung, die zur Anwendung in Form kleinster Tropfen geeignet ist.

II. 20 Gewichtsteile der Verbindung Nr. 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in Wasser erhält man eine wäßrige Dispersion.

III. 20 Gewichtsteile der Verbindung Nr. 15 werden in einer Mischung gelöst, die aus 40 Gewichtsteilen Cyclohexanon, 30 Gewichtsteilen Isobutanol, 20 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gewichtsteile der Verbindung Nr. 17 werden in einer Mischung gelöst, die aus 25 Gewichtsteilen Cyclohexanol, 65 Gewichtsteilen einer Mineralölfraktion vom Siedepunkt 210 bis 280°C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gewichtsteile der Verbindung Nr. 20 werden den mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer

Hammermühle vermahlen. Durch feines Verteilen der Mischung in Wasser erhält man eine Spritzbrühe.

VI. 3 Gewichtsteile der Verbindung Nr. 1 werden mit 97 Gewichtsteilen feinteiligem Kaolin innig vermischt. Man erhält auf diese Weise ein Stäubemittel, das 3 Gew.% des Wirkstoffs enthält.

VII. 30 Gewichtsteile der Verbindung Nr. 22 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gewichtsteile der Verbindung Nr. 18 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion. Durch Verdünnen mit Wasser erhält man eine wäßrige Dispersion.

IX. 20 Teile der Verbindung Nr. 2 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden oder auch mit Düngemitteln vermischt und ausgebracht werden. Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen kombiniert werden können, soll die Kombinationsmöglichkeiten erläutern, nicht aber einschränken.

Fungizide, die mit den erfindungsgemäßen Verbindungen kombiniert werden können, sind beispielsweise:

Schwefel,

Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N′-ethylen-bis-dithiocarbamat)
Ammoniak-Komplex von Zink-(N,N′-propylen-bis-dithiocarbamat)
Zink-(N,N′-propylen-bis-dithiocarbamat)
N,N′-Polypropylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester,

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol
2,3-Dicyano-1,4-dithiaanthrachinon
2-Thio-1,3-dithio-(4,5,6)-chinoxalin
1-(Butylcarbamoyl)-2-benzimidazol-carbaminsäuremethylester
2-Methoxycarbonylamino-benzimidazol
2-(Furyl-(2)-benzimidazol
2-(Thiazolyl-(4)-benzimidazol
N-(1,1,2,2-Tetrachlorethylthio)-tetrahydrophthalimid
N-Trichlormethylthio-tetrahydrophthalimid
N-Trichlormethylthio-phthalimid
N-Dichlorfluormethylthio-N′,N′-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon

Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid
2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1[2-(2,4-Dichlorphenyl)-4-n-propyl-1,3-dioxolan-2-yl-ethyl]-1-H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hyroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-gluataramid
Hexachlorbenzol
DL-Methyl-N-(2,6-dimethyl-phenyl)-N-furoyl(2)-alaninat,
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl(-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid.
2-Cyano-N-(ethylaminocarbonyl)-2-methoximino)-acetamid
1-(2-(2,4-Dichlorphenyl)-pentyl)-1H-1,2,4-triazol
2,4-Difluor-alpha-(1H-1,2,4-triazolyl-1-methyl)-benzhydrylalkohol

Für die folgenden Versuche wurde als Vergleich der bekannte Wirkstoff 3-(4-Chlorphenyl)-1-(3-pyridinyl)-2-propen-1-on (A) verwendet.

## Anwendungsbeispiel 1

### Wirksamkeit gegen Weizenmehltau

Blätter von in Töpfen gewachsenen Weizenkeimlingen der Sorte Frühgold werden mit wäßriger Spritzbrühe, die 80 % Wirkstoff und 20 % Emulgiermittel in der Trockensubstanz enthält, besprüht und 24 Stunden nach dem Antrocknen des Spritzbelages mit Oidien (Sporen) des Weizenmehltaus (Erysiphe graminis var. tritici) bestäubt. Die Versuchspflanzen werden anschließend im Gewächshaus bei Temperaturen zwischen 20 und 22° C und 75 bis 80 % relativer Luftfeuchtigkeit aufgestellt. Nach 7 Tagen wird das

Ausmaß der Mehltauentwicklung ermittelt.

Das Ergebnis des Versuches zeigt, daß beispielsweise die Verbindungen Nr. 1, 2, 15, 17, 18 und 20 bei der Anwendung als 0,025%ige Spritzbrühe eine bessere fungizide Wirkung haben (z.B. 97 %) als der bekannte Wirkstoff A (60 %).

Anwendungsbeispiel 2

Wirksamkeit gegen Gurkenmehltau

Blätter von in Töpfen gewachsenen Gurkenkeimlingen der Sorte "Chinesische Schlange" werden im Zweiblattstadium mit einer Sporensuspension des Gurkenmehltaus besprüht. Nach etwa 20 Stunden werden die Versuchspflanzen mit wäßriger Spritzbrühe, die 80 % Wirkstoff und 20 % Emulgiermittel in der Trockensubstanz enthält, bis zur Tropfnässe besprüht. Nach dem Antrocknen des Spritzbelages werden sie anschließend im Gewächshaus bei Temperaturen zwischen 20 und 22° C und 70 bis 80 % relativer Luftfeuchtigkeit aufgestellt. Zur Beurteilung der Wirksamkeit der neuen Stoffe wird das Ausmaß der Pilzentwicklung nach 8 Tagen ermittelt.

Das Ergebnis des Versuches zeigt, daß beispielsweise die Verbindungen Nr. 1, 2, 15, 16, 18, 20 und 22 bei der Anwendung als 0,025%ige Spritzbrühe eine bessere fungizide Wirkung haben (z.B. 100 %) als der bekannte Wirkstoff A (70 %).

Anwendungsbeispiel 3

Wirksamkeit gegen Weizenbraunrost

Blätter von in Töpfen gewachsenen Weizensämlingen der Sorte "Frühgold" werden mit Sporen des Braunrostes (Puccinia recondita) bestäubt. Danach werden die Töpfe für 24 Stunden bei 20 bis 22° C in eine Kammer mit hoher Luftfeuchtigkeit (90 bis 95 %) gestellt. Während dieser Zeit keimen die Sporen aus und die Keimschläuche dringen in das Blattgewebe ein. Die infizierten Pflanzen werden anschließend mit wäßrigen Spritzbrühen, die 80 % Wirkstoff und 20 % Emulgiermittel in der Trockensubstanz enthalten, tropfnaß gespritzt. Nach dem Antrocknen des Spritzbelages werden die Versuchspflanzen im Gewächshaus bei Temperaturen zwischen 20 und 22° C und 65 bis 70 % relativer Luftfeuchte aufgestellt. Nach 8 Tagen wird das Ausmaß der Rostpilzentwicklung auf den Blättern ermittelt.

Das Ergebnis des Versuches zeigt, daß beispielsweise die Verbindungen Nr. 2, 16, 18, 20 und 22 bei der Anwendung als 0,025%ige Spritzbrühe eine bessere fungizide Wirkung haben (z.B. 97 %) als der bekannte Wirkstoff A (50 %).

Anwendungsbeispiel 4

Wirksamkeit gegen Botrytis cinerea an Paprika

Paprikasämlinge der Sorte "Neusiedler Ideal Elite" werden, nachdem sich 4-5 Blätter gut entwickelt haben, mit wäßrigen Suspensionen, die 80 % Wirkstoff und 20 % Emulgiermittel in der Trockensubstanz enthalten, tropfnaß gespritzt. Nach dem Antrocknen des Spritzbelages werden die Pflanzen mit einer Konidienaufschwemmung des Pilzes Botrytis cinerea besprüht und bei 22-24° C in eine Kammer mit hoher Luftfeuchtigkeit gestellt. Nach 5 Tagen hat sich die Krankheit auf den unbehandelten Kontrollpflanzen so stark entwickelt, daß die entstandenen Blattnekrosen den überwiegenden Teil der Blätter bedecken.

Das Ergebnis des Versuches zeigt, daß beispielsweise die Verbindungen Nr. 1, 2, 16, 18, 20 und 22 bei der Anwendung als 0,05%ige Spritzbrühe eine bessere fungizide Wirkung haben (z.B. 97 %) als der bekannte Wirkstoff A (60 %).

Anwendungsbeispiel 5

Wirksamkeit gegen Apfelschorf

Junge Blätter von in Töpfen gewachsenen Apfelsämlingen der Sorte "Golden Delicious" werden mit wäßriger Spritzbrühe, die 80 % Wirkstoff und 20 % Emulgiermittel in der Trockensubstanz enthält, tropfnaß gespritzt. Nach dem Antrocknen des Spritzbelages werden die Versuchspflanzen mit einer Sporensuspension des Apfelschorfs (Venturia inaequalis) besprüht. Anschließend werden die besprühten Pflanzen in einer Klimakammer bei 20 und 22 °C und 95 % relativer Luftfeuchtigkeit für 10 Tage aufgestellt. Dann wird das Ausmaß der Pilzentwicklung auf den Blättern ermittelt.

Das Ergebnis zeigt, daß beispielsweise die Verbindung Nr. 2 bei der Anwendung als 0,0075 und 0,00375%ige Spritzbrühe eine gute fungizide Wirkung hat (z.B. 97 %).

## Ansprüche

**1.** Pyridine der Formel

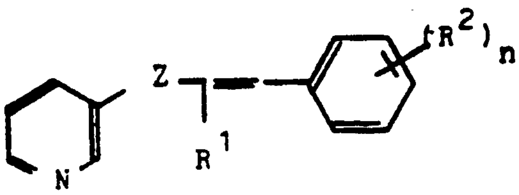

(I),

in der

$R^1$ für $C_1$-$C_8$-Alkyl, $C_3$-$C_7$-Cycloalkyl, gegebenenfalls durch Halogen, $C_1$-$C_4$-Alkyl, $C_1$-$C_4$-Alkoxy, Phenyl, Nitro substituiertes Phenyl

$R^2$ für Wasserstoff, Halogen, $C_1$-$C_5$-Alkoxy, $C_1$-$C_5$-Alkyl, Phenyl,

n für die Zahlen 1, 2, 3,

Z für C = O oder CHOH stehen

und die pflanzenphysiologisch verträglichen Säureadditionssalze dieser Verbindungen.

**2.** Verfahren zur Herstellung eines Pyridins gemäß Anspruch 1, dadurch gekennzeichnet, daß man ein Pyridylketon der Formel II

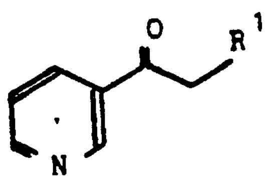

II,

in der $R^1$ die obengenannte Bedeutung hat, umsetzt mit einem Benzaldehyd der Formel

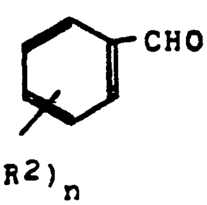

III,

in der $R^2$ und n die in Anspruch 1 genannten Bedeutungen haben und gegebenenfalls das entstandene Pyridylketon der Formel IV

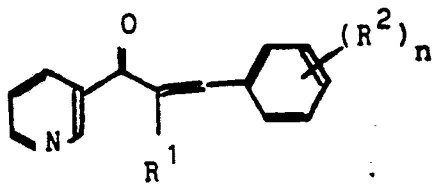

IV,

in der $R^1$, $R^2$ und n die in Anspruch 1 genannten Bedeutungen haben, zum Pyridylcarbinol der Formel V reduziert.

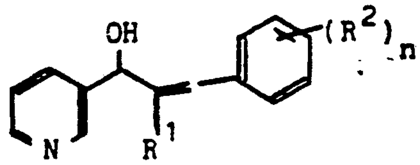

V

3. Fungizides Mittel, enthaltend einen inerten Trägerstoff und ein Pyridin der Formel (I) gemäß Anspruch 1.

4. Verfahren zur Bekämpfung von Pilzen, dadurch gekennzeichnet , daß man die Pilze oder die von Pilzbefall bedrohten Materialien, Pflanzen, Böden oder Saatgüter behandelt mit einer fungizid wirksamen Menge eines Pyridins der Formel (I) gemäß Anspruch 1.

5. 2-Butyl-3-(2,4-dichlorbenzyl)-1-pyridin-3-yl-2-propen-1-ol.

6. 2-Butyl-3-(4-fluorbenzyl)-1-pyridin-3-yl-2-propen-1-ol.

7. 2-Butyl-3-(4-methylbenzyl)-1-pyridin-3-yl-2-propen-1-ol.

**Claims**

1. A pyridine of the formula

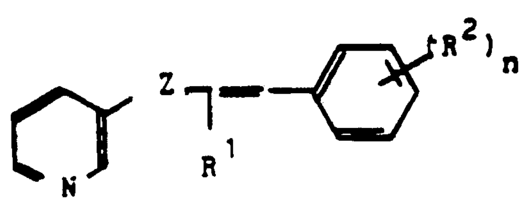

(I)

where $R^1$ is $C_1$-$C_8$-alkyl or $C_3$-$C_7$-cycloalkyl or is phenyl which is unsubstituted or substituted by halogen, $c_1$-$c_4$-alkyl, $C_1$-$C_4$-alkoxy, phenyl or nitro, $R^2$ is hydrogen, halogen, $C_1$-$C_6$-alkoxy, $C_1$-$C_6$-alkyl or phenyl, n is 1, 2 or 3 and Z is C=O or CHOH, and the phytophysiologically tolerated addition salts of this compound with acids.

2. A process for the preparation of a pyridine as claimed in claim 1, wherein a pyridylketone of the formula II

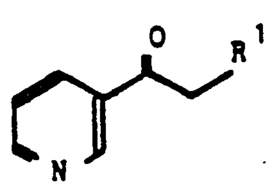

II

where $R^1$ has the abovementioned meanings, is reacted with a benzaldehyde of the formula

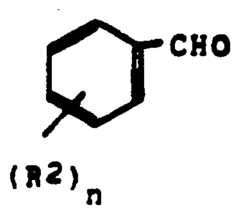

III

where $R^2$ and n have the meanings stated in claim 1, and, if required, the resulting pyridylketone of the formula IV

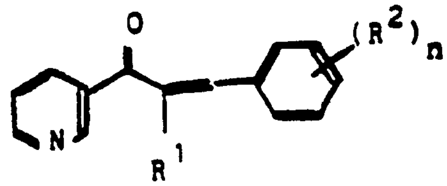

IV

where $R^1$, $R_2$ and n have the meanings stated in claim 1, is reduced to a pyridylcarbinol of the formula V

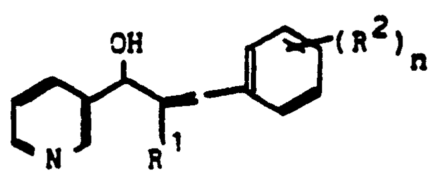

V.

3. A fungicide containing an inert carrier and a pyridine of the formula (I) as claimed in claim 1.

4. A method for controlling fungi, wherein the fungi or the materials, plants, soils or seeds threatened by fungal attack are treated with a fungicidal amount of a pyridine of the formula (I) as claimed in claim 1.

5. 2-Butyl-3-(2,4-dichlorobenzyl)-1-pyridin-3yl-2 propen-1-ol.

6. 2-Butyl-3-(4-fluorobenzyl)-1-pyridin-3-yl-2-propen-1-ol.

7. 2-Butyl-3-(4-methylbenzyl)-1-pyridin-3-yl-2-propen-1-ol.

## Revendications

1. Pyridines de formule

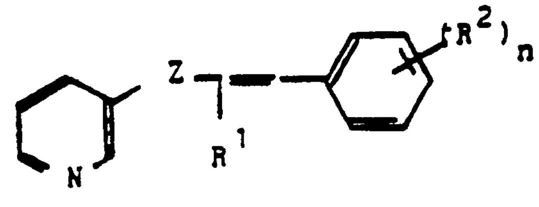

(I)

dans laquelle
$R^1$ représente un groupe alkyle en C 1-C 8, cycloalkyle en C 3-C 7, phényle, éventuellement substitué par halogène, alkyle en C 1-C 4, alcoxy en C 1-C 4, phényle, nitro,
$R^2$ représente l'hydrogène, un halogène, un groupe alcoxy en C 1-C 5, alkyle en C 1-C 5, phényle,

n est égal à 1, 2, 3,
Z représente C = O ou CHOH,
et les sels de ces composés formés par addition avec des acides et tolérés par les végétaux.

**2.** Procédé de préparation d'une pyridine selon la revendication 1, caractérisé en ce que l'on fait réagir une pyridylcétone de formule II

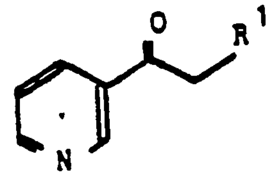

II,

dans laquelle $R^1$ a les significations indiquées cidessus, avec un benzaldéhyde de formule

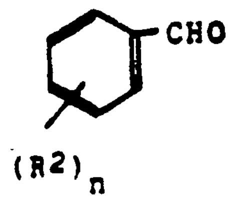

III,

dans laquelle R2 et n ont les significations indiquées dans la revendication 1, ce qui donne une pyridylcétone de formule IV

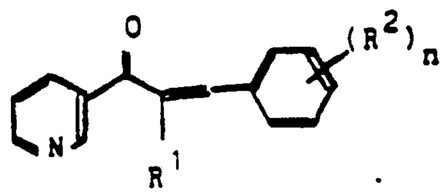

IV,

dans laquelle $R^1$, $R^2$ et n ont les significations indiquées dans la revendication 1, qu'on réduit le cas échéant en le pyridylcarbinol de formule V

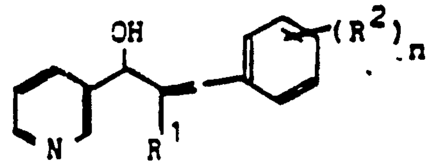

V

**3.** Produit fongicide contenant un véhicule inerte et une pyridine de formule I selon la revendication 1.

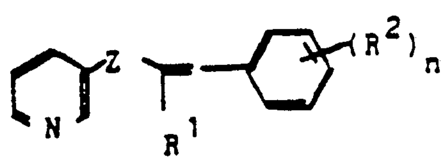

I,

**4.** Procédé pour combattre les mycètes, caractérisé en ce que l'on traite les mycètes ou les matériaux, végétaux, sols ou semences menacés d'une attaque par les mycètes par une quantité fongicide

efficace d'une pyridine de formule I selon la revendication 1.

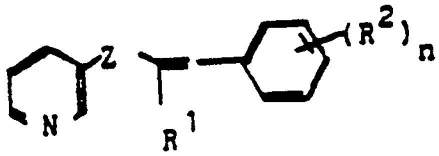 I,

**5.** Le 2-butyl-3-(2,4-dichlorobenzyl)-1-pyridine-3-yl-2-propèn-1-ol.

**6.** Le 2-butyl-3-(4-fluorobenzyl)-1-pyridine-3-yl-2-propèn-1-ol.

**7.** Le 2-butyl-3-(4-méthylbenzyl)-1-pyridine-3-yl-2-propèn-1-ol.